# EUROPEAN PATENT APPLICATION

(11) **EP 4 834 201 A1**
(43) Date of publication of application: **04.11.2026**
(21) Application number: 24911846.4
(22) Date of filing: 23.08.2024
(51) Int. Cl.: A61B 3/10

(54) **OPHTHALMIC SYSTEM**

(30) Priority: 26.12.2023 JP 2023220118
(71) Applicant: Topcon Corporation, Tokyo 174-8580 (JP)
(72) Inventor: OMIYA, Takeshi, Tokyo 174-8580 (JP)
(74) Representative: Louis Pöhlau Lohrentz
(86) International application number: PCT/JP2024/029959
(87) International publication number: WO 2025/141951

(57) **Abstract**

To provide an ophthalmic system that enables an eye examination using an ophthalmic apparatus to be performed under the supervision of an examiner, an ophthalmic system (1) includes an ophthalmic apparatus (10) that is used in an eye examination and includes a subject microphone (17) that is configured to capture audio; and a subject speaker (16) that is configured to output audio; and a plurality of host apparatuses (30) that are operated by an examiner and include an examiner microphone (34) that is configured to capture audio; an examiner speaker (33) that is configured to output audio; and an examiner monitor (32) that is viewable by the examiner. The ophthalmic apparatus (10) and the plurality of host apparatuses (30) are configured to communicate with one another. Each of the host apparatuses (30) is configured to cause a result of the eye examination to be displayed on the examiner monitor (32) and to cause the audio captured by the subject microphone (17) to be output through the examiner speaker (33). The ophthalmic apparatus (10) is configured to cause the audio captured by the examiner microphone (34) to be output through the subject speaker (16).

## Description

### [Technical Field]

The present invention relates to an ophthalmic system.

### [Background Art]

A conventional ophthalmic system is known which allows an examiner to remotely operate an ophthalmic apparatus using a host apparatus via communication networks such as the Internet (for example, refer to Patent Literature 1). In addition, an ophthalmic system is known in which, when an examiner remotely operates an ophthalmic apparatus, the examiner may issue audio instructions to a subject using a microphone provided in a host apparatus and a speaker provided in the ophthalmic apparatus (for example, refer to Patent Literature 2).

### [Citation List]

### [Patent Literature]

[Patent Literature 1] JP 2022-073686 A
[Patent Literature 2] JP 2023-039505 A

### [Summary of Invention]

### [Technical Problem]

In conventional ophthalmic systems, a single ophthalmic apparatus communicably connects to a single host apparatus. An examiner (such as an ophthalmologist, an optometrist, or an orthoptist) who operates the host apparatus monitors an eye examination, including controlling the ophthalmic apparatus connected to the host apparatus. Therefore, when the examiner is no longer present, an eye examination using the ophthalmic apparatus can no longer be performed under the supervision of the examiner, making it difficult to conduct appropriate eye examinations.

The present invention has been made in view of the problem described above and an object thereof is to provide an ophthalmic system that allows eye examinations using an ophthalmic apparatus to be performed under the supervision of an examiner.

### [Solution to Problem]

An ophthalmic system according to the present invention includes an ophthalmic apparatus that is used in an eye examination, the ophthalmic apparatus including a subject microphone that is configured to capture audio; and a subject speaker that is configured to output audio; and a plurality of host apparatuses that are operated by an examiner, the plurality of host apparatuses including an examiner microphone that is configured to capture audio; an examiner speaker that is configured to output audio; and an examiner monitor that is viewable by the examiner. The ophthalmic apparatus and the plurality of host apparatuses are configured to communicate with one another. Each of the host apparatuses is configured to cause a result of the eye examination to be displayed on the examiner monitor and to cause the audio captured by the subject microphone to be output through the examiner speaker. The ophthalmic apparatus is configured to cause the audio captured by the examiner microphone to be output through the subject speaker.

### [Advantageous Effect of Invention]

The ophthalmic system according to the present invention allows an eye examination using an ophthalmic apparatus to be performed under the supervision of an examiner.

### [Brief Description of Drawings]

[FIG. 1] FIG. 1 is a schematic configuration diagram illustrating an example of an ophthalmic system according to a first embodiment.
[FIG. 2] FIG. 2 is a block diagram illustrating a configuration of a control system in the ophthalmic system.
[FIG. 3] FIG. 3 is a flow chart illustrating a flow of processing of an eye examination in the ophthalmic system according to the first embodiment.
[FIG. 4] FIG. 4 is a sub-flow chart illustrating login signal reception processing according to the first embodiment.
[FIG. 5] FIG. 5 is a sub-flow chart illustrating remote operation configuration processing according to the first embodiment.
[FIG. 6] FIG. 6 is a sub-flow chart illustrating eye examination processing according to the first embodiment.
[FIG. 7] FIG. 7 is a sub-flow chart illustrating examination guidance processing according to the first embodiment.
[FIG. 8] FIG. 8 is a sub-flow chart illustrating examination execution processing according to the first embodiment.
[FIG. 9] FIG. 9 is an explanatory diagram illustrating a display example of an examiner monitor according to the first embodiment.
[FIG. 10] FIG. 10 is an explanatory diagram illustrating a display example of a subject monitor according to the first embodiment.
[FIG. 11] FIG. 11 is a schematic configuration diagram illustrating an example of an ophthalmic system according to a second embodiment.
[FIG. 12] FIG. 12 is a sub-flow chart illustrating login signal reception processing according to the second embodiment.
[FIG. 13] FIG. 13 is a sub-flow chart illustrating remote operation configuration processing according to the second embodiment.

### [Description of Embodiments]

Embodiments for implementing the ophthalmic system according to the present invention are described below based on first and second embodiments shown in the drawings.

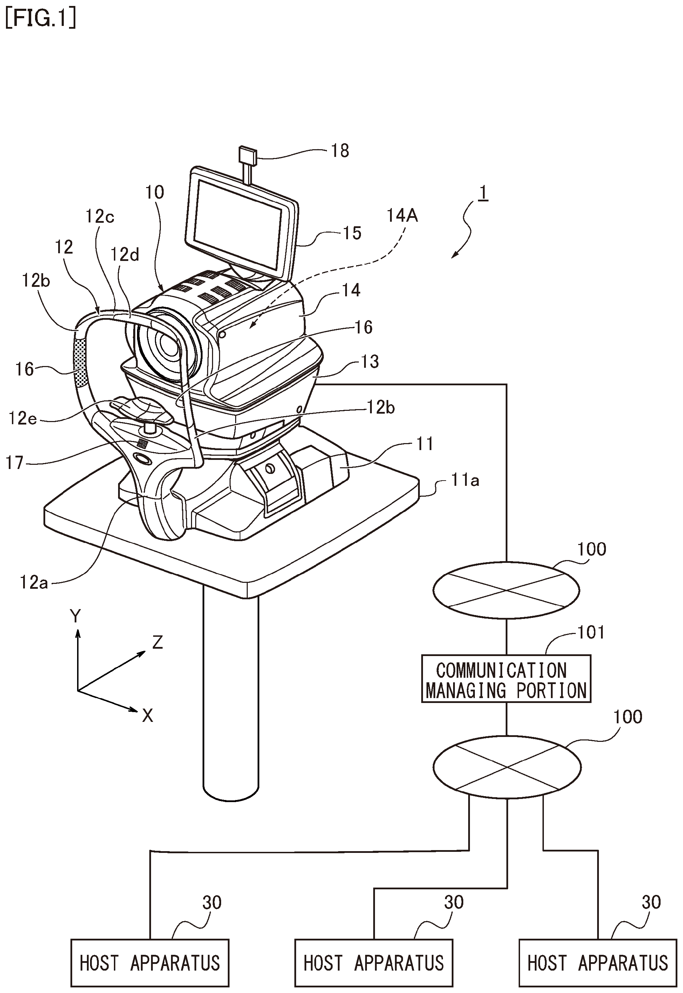

(First Embodiment) An ophthalmic system 1 according to a first embodiment is described below with reference to FIG. 1. As shown in FIG. 1, the ophthalmic system 1 according to the first embodiment includes one ophthalmic apparatus 10, a plurality of host apparatuses 30, which include three host apparatuses in this example, a communication network 100, and a communication managing portion 101.

The ophthalmic apparatus 10 is at a remote location, away from the plurality of host apparatuses 30. For example, the ophthalmic apparatus 10 is installed at a location such as an eyewear store or a mobile clinic, while the plurality of host apparatuses 30 are installed at one or more hospitals where examiners are based. A subject is present at a location where the ophthalmic apparatus 10 is installed, and the examiners are present at a location where the host apparatuses 30 are installed. Consequently, the examiners are not located at the same location as the subject. The plurality of host apparatuses 30 may be located at separate locations or may be adjacent (in close proximity) to one another. An examiner refers to a person qualified to conduct examinations and make diagnoses using the ophthalmic apparatus 10 such as an ophthalmologist, an optometrist, or an orthoptist.

The communication network 100 is a network such as the Internet or a local area network (LAN).

The ophthalmic apparatus 10 and the plurality of host apparatuses 30 are each connected to the communication network 100 and are capable of communicating with one another via the communication network 100 and the communication managing portion 101. The communication managing portion 101 manages the communication status between the ophthalmic apparatus 10 and the plurality of host apparatuses 30.

The ophthalmic apparatus 10 according to the first embodiment is a multifunctional instrument capable of measuring intraocular pressure, ocular refraction, and corneal curvature of an eye, and is used for examining the eye. The ophthalmic apparatus 10 measures various ocular characteristics of the subject, such as intraocular pressure, ocular refraction, and corneal curvature (corneal shape) of the eye. The ophthalmic apparatus 10 according to the first embodiment is remotely operated by one of the host apparatuses 30. Results of the eye examination by the ophthalmic apparatus 10 are displayed so as to be viewable on all of the plurality of host apparatuses 30.

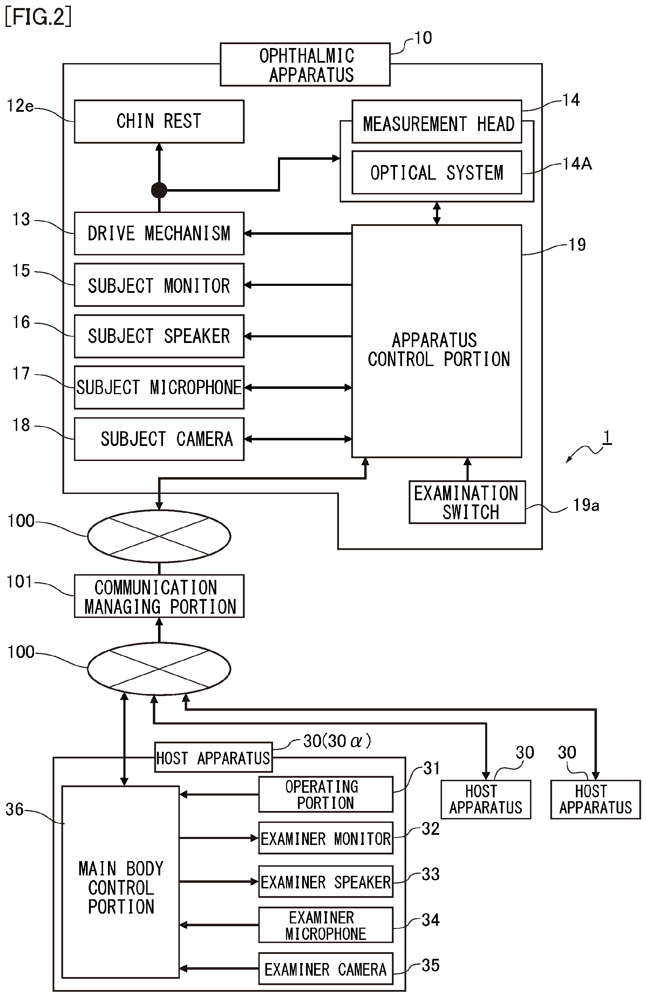

As shown in FIG. 1, the ophthalmic apparatus 10 includes a base 11, a face support 12, a drive mechanism 13, a measurement head 14, a subject monitor 15, subject speakers 16, a subject microphone 17, a subject camera 18, and an apparatus control portion 19 (refer to FIG. 2). Among the mutually orthogonal X-, Y-, and Z-directions (the three axial directions) shown in the diagram, the Y-direction corresponds to the up-down direction. The Z-direction is the anteroposterior direction (working distance direction) parallel to the forward direction approaching the subject's eye (not illustrated) and the rearward direction away from the subject. In addition, the X-direction is the left-right direction perpendicular to both the vertical and anteroposterior directions.

The base 11 is provided with the drive mechanism 13 for supporting the face support 12 and the measurement head 14, and is installed on an eye examination table 11a.

The face support 12 is arranged in front of the drive mechanism 13. The face support 12 includes a support pillar 12a, a pair of side frame portions 12b, an upper frame portion 12c, a forehead rest 12d, and a chin rest 12e.

The support pillar 12a extends upward and downward from the base 11. The pair of side frame portions 12b are aligned in the X-axis direction and extend upward from the support pillar 12a. The upper frame portion 12c spans across upper ends of the side frame portions 12b. The forehead rest 12d is provided in the center of the upper frame portion 12c. The chin rest 12e is arranged between the side frame portions 12b and protrudes from the support pillar 12a.

During an eye examination, the subject sits in front of the ophthalmic apparatus 10, rests their chin on the chin rest 12e, and presses their forehead against the forehead rest 12d to fix the position of their face. The chin rest 12e can be moved in the Y-direction relative to the base 11 by a drive mechanism (not illustrated).

The drive mechanism 13 corresponds to a relative movement mechanism of the ophthalmic apparatus 10. For example, the drive mechanism 13 is implemented by an actuator (not illustrated) such as a motor. The drive mechanism 13 is driven based on control signals (control commands) from the apparatus control portion 19. The drive mechanism 13 can move the chin rest 12e in the Y-direction based on a control signal. The drive mechanism 13 also moves the measurement head 14 in each of the X-, Y-, and Z-directions relative to the base 11 based on control signals.

A known optical system 14A for observation and capturing images (refer to FIG. 2) is provided inside the measurement head 14. The optical system 14A is, for example, an auto refractor keratometer. The optical system 14A is driven by control signals from the apparatus control portion 19 to observe and capture various ocular characteristics of the subject for eye examinations. Information acquired by the optical system 14A including the results of the eye examination and observation images (hereinafter referred to as "examination result information") is input to the apparatus control portion 19.

The subject monitor 15 is, for example, a monitor such as a liquid crystal monitor or a touch screen monitor. The subject monitor 15 is mounted on the rear side of the measurement head 14. The subject monitor 15 displays image-capture information (hereinafter referred to as "examiner image-capture information") obtained by an examiner camera 35 (to be described later) based on control signals from the apparatus control portion 19. The subject monitor 15 may also display examination result information. Furthermore, when the subject monitor 15 is a touch screen monitor, the subject monitor 15 may display screens such as operation menu screens for various operations and accept operation input for each of the operation menu screens. In this case, an operator beside the subject or the subject themselves can directly operate the ophthalmic apparatus 10.

The subject speakers 16 are built into the pair of side frame portions 12b to be positioned near the subject's left and right ears during an eye examination. The subject speakers 16 output audio including audio information (hereinafter referred to as "examiner audio information") captured by an examiner microphone 34 (to be described later) based on control signals from the apparatus control portion 19.

The subject microphone 17 is built into an upper part of the support pillar 12a and arranged below the chin rest 12e. The subject microphone 17 captures audio including the subject's voice based on control signals from the apparatus control portion 19. Audio information (hereinafter referred to as "subject audio information") captured by the subject microphone 17 is input to the apparatus control portion 19.

The subject camera 18 is positioned so as to capture the subject in an examination posture and, in this example, the subject camera 18 is provided on top of the subject monitor 15. The subject camera 18 captures images based on control signals from the apparatus control portion 19. Image-capture information (hereinafter referred to as "subject image-capture information") obtained by the subject camera 18 is input to the apparatus control portion 19.

The subject camera 18 is capable of capturing images of the subject in the examination posture as well as of the subject standing in front of the face support 12. Furthermore, the subject camera 18 according to the first embodiment is capable of capturing images of the area surrounding the ophthalmic apparatus 10, specifically the area in front of the subject camera 18. Thus, the subject image-capture information includes images of the subject arriving for an eye examination and the subject's face during the examination. In addition, the examination postures include a posture in which the subject is seated in a chair or stool (not illustrated) provided in front of the ophthalmic apparatus 10, a posture in which the subject rests their chin on the chin rest 12e, and a posture in which the subject rests their chin on the chin rest 12e and their forehead against the forehead rest 12d. As described later, the subject image-capture information displayed on the examiner monitor 32 allows the examiner to observe the subject's condition before, during, and after the eye examination by looking at the examiner monitor 32. The examiner can also use the display on the examiner monitor 32 to adjust the position of the ophthalmic apparatus 10 relative to the subject.

The apparatus control portion 19 includes a processing circuit constituted by components including various processors and memories (storage portions). The various processors include central processing units (CPUs), graphics processing units (GPUs), application-specific integrated circuits (ASICs), and programmable logic devices [such as simple programmable logic devices (SPLDs), complex programmable logic devices (CPLDs), and field-programmable gate arrays (FPGAs)]. Various controls (functions) of the apparatus control portion 19 may be realized by one processor or a plurality of similar or different processors.

The apparatus control portion 19 is activated when a power switch (not illustrated) is turned on by a person such as the subject or the operator, and sets the ophthalmic apparatus 10 to sleep mode until remote operation by an operation host apparatus 30α (to be described later) begins. The term "sleep mode" refers to a mode in which power supply to mechanisms of the ophthalmic apparatus 10 other than the apparatus control portion 19 is stopped or restricted, thereby suspending some functions of the ophthalmic apparatus 10. In the first embodiment, when the sleep mode is on, operations of at least the chin rest 12e, the drive mechanism 13, the measurement head 14, the subject monitor 15, the subject speakers 16, the subject microphone 17, and the subject camera 18 are suspended.

When an examination switch 19a is turned on, the apparatus control portion 19 outputs a request signal to the communication managing portion 101. The "request signal" is a signal indicating that a person such as the subject has requested an eye examination. In addition, the apparatus control portion 19 outputs information such as examination result information, subject audio information, and subject image-capture information to the host apparatuses 30 via the communication managing portion 101.

The apparatus control portion 19 also receives information such as operation information, examiner audio information, and examiner image-capture information from the host apparatuses 30 via the communication managing portion 101. Based on the operation information, the apparatus control portion 19 reads a control program stored in a memory (not illustrated) and executes the program. Accordingly, the apparatus control portion 19 outputs predetermined control signals to the ophthalmic apparatus 10 including the drive mechanism 13 and the optical system 14A to control operations so that the ophthalmic apparatus 10 transitions between modes and examines eyes. The apparatus control portion 19 also outputs a control signal to the subject monitor 15 to display the examiner image-capture information. The apparatus control portion 19 also outputs a control signal to the subject speakers 16 to reproduce the examiner audio information. The apparatus control portion 19 also outputs a control signal to the subject microphone 17 to capture audio. The apparatus control portion 19 also outputs a control signal to the subject camera 18 to capture images.

Furthermore, when a sleep signal is input as operation information, the apparatus control portion 19 causes the ophthalmic apparatus 10 to transition to the sleep mode. When a wake signal is input as operation information, the apparatus control portion 19 causes the ophthalmic apparatus 10 to transition to an examination mode.

Each of the host apparatuses 30 is constituted by, for example, a personal computer or a tablet terminal. Each of the host apparatuses 30 is operated by a different examiner and is used when the examiner remotely operates the ophthalmic apparatus 10 and views examination results. Each of the host apparatuses 30 includes an operating portion 31, an examiner monitor 32, an examiner speaker 33, an examiner microphone 34, an examiner camera 35, and a main body control portion 36.

The operating portion 31 is constituted by, for example, a keyboard and a mouse. The operating portion 31 is operated by the examiner to input instructions for outputting signals such as login signals, operation authorization signals, operation handover signals, and examination completion signals, operation information for remotely operating the ophthalmic apparatus 10, and instructions for outputting various control signals. The operation information and instructions for outputting the various signals received from the operating portion 31 are input to the main body control portion 36.

The "login signal" is a signal indicating that the examiner can remotely operate a host apparatus 30. The "operation authorization signal" is a signal notifying authorization for remote operation of the host apparatus 30 by the examiner. The "operation handover signal" is a signal indicating a handover request for the operation host apparatus 30α (to be described later). The "examination completion signal" is a signal indicating the completion of an eye examination by the operation host apparatus 30α (to be described later).

The examiner monitor 32 is constituted by, for example, a liquid crystal display. In the ophthalmic system 1 according to the first embodiment, the examiner monitor 32 may be constituted by a touch screen monitor, which also serves as the operating portion 31. Based on control signals from the main body control portion 36, the examiner monitor 32 displays examination result information received from the ophthalmic apparatus 10, subject image-capture information, and examiner image-capture information from the other host apparatuses 30.

The examiner speaker 33 and the examiner microphone 34 are constituted by, for example, a headset (headphone with a microphone). The examiner speaker 33 outputs audio including subject audio information and examiner audio information from the other host apparatuses 30 based on control signals from the main body control portion 36. The examiner microphone 34 captures audio including the examiner's voice (examiner audio information) based on control signals from the main body control portion 36. The examiner microphone 34 provides the examiner audio information to the main body control portion 36.

The examiner camera 35 is arranged at a position where at least the examiner's face can be captured. The examiner camera 35 captures images based on control commands from the main body control portion 36. The examiner camera 35 provides the examiner image-capture information to the main body control portion 36. The examiner image-capture information desirably contains images of the examiner's face.

The main body control portion 36 includes a processing circuit constituted by components including various processors and memories (storage portions) in a similar manner to the apparatus control portion 19. The various controls (functions) of the main body control portion 36 may be realized by one processor or a plurality of similar or different processors.

The main body control portion 36 is activated when a power switch (not illustrated) is turned on by a person such as the examiner. When an instruction for outputting a login signal is input by an operation of the operating portion 31, the main body control portion 36 outputs the login signal to the communication managing portion 101. When an instruction for outputting an operation authorization signal is input by an operation of the operating portion 31, the main body control portion 36 outputs the operation authorization signal to the communication managing portion 101. When an instruction for outputting an operation handover signal is input by an operation of the operating portion 31, the main body control portion 36 outputs the operation handover signal to the communication managing portion 101. When an instruction for outputting an examination completion signal is input by an operation of the operating portion 31, the main body control portion 36 outputs the examination completion signal to the communication managing portion 101. Furthermore, the main body control portion 36 outputs information such as operation information, examiner audio information, and examiner image-capture information to the ophthalmic apparatus 10 via the communication managing portion 101 in response to operations of the operating portion 31.

The main body control portion 36 also receives remote operation disabled signals, request standby signals, and operation inquiry signals from the communication managing portion 101. The main body control portion 36 receives examination result information, subject audio information, and subject image-capture information from the ophthalmic apparatus 10 via the communication managing portion 101. The main body control portion 36 receives examiner audio information and examiner image-capture information from the other host apparatuses 30 via the communication managing portion 101. When the communication managing portion 101 authorizes remote operation of the ophthalmic apparatus 10, the main body control portion 36 receives information on the statuses of the optical system 14A and the drive mechanism 13 from the ophthalmic apparatus 10 via the communication managing portion 101 in addition to the information described above.

Based on operations of the operating portion 31 by the examiner, the main body control portion 36 outputs control signals to the examiner monitor 32, thereby causing the examiner monitor 32 to display information such as examination result information, subject image-capture information, and examiner image-capture information from the other host apparatuses 30. The main body control portion 36 outputs control signals to the examiner speaker 33, thereby causing the examiner speaker 33 to output subject audio information and examiner audio information from the other host apparatuses 30. The main body control portion 36 outputs a control signal to the examiner microphone 34, thereby causing the examiner microphone 34 to capture audio. The main body control portion 36 outputs a control signal to the examiner camera 35, thereby causing the examiner camera 35 to capture images.

The communication managing portion 101 manages and controls the communication status between the ophthalmic apparatus 10 and the plurality of host apparatuses 30 as well as the communication status among the plurality of host apparatuses 30. The communication managing portion 101 is implemented by a computer or a similar device separate from the host apparatuses 30. The communication managing portion 101 is communicatively connected to the ophthalmic apparatus 10 and the plurality of host apparatuses 30 via the communication network 100 so as to be capable of communicating with one another.

The communication managing portion 101 receives a login signal from each of the host apparatuses 30. The communication managing portion 101 outputs a remote operation disabled signal or a request standby signal to the host apparatus 30 having output the login signal.

The communication managing portion 101 also receives a request signal from the ophthalmic apparatus 10. The communication managing portion 101 outputs an operation inquiry signal to the plurality of host apparatuses 30 having output the login signal. As a result, the communication managing portion 101 authorizes the host apparatus 30 having output an operation authorization signal to remotely operate the ophthalmic apparatus 10 and controls communication so that the ophthalmic apparatus 10 is remotely operated based on control signals from the host apparatus 30. Specifically, the communication managing portion 101 allows input of operation information to the ophthalmic apparatus 10 from the host apparatus authorized to remotely operate the ophthalmic apparatus 10 (hereinafter referred to as "operation host apparatus 30α") and blocks input of operation information to the ophthalmic apparatus 10 from the other host apparatuses 30. The communication managing portion 101 maintains authorization for remote operation by the operation host apparatus 30α until the operation host apparatus 30α outputs an examination completion signal or an operation handover signal.

The communication managing portion 101 allows the examination result information, the subject audio information, and the subject image-capture information output from the ophthalmic apparatus 10 to be input to all host apparatuses 30 having output a login signal. The communication managing portion 101 allows the examiner audio information and the examiner image-capture information output from all host apparatuses 30 having output a login signal to be input to the ophthalmic apparatus 10.

Furthermore, the communication managing portion 101 allows the examiner audio information and the examiner image-capture information to be mutually input among all host apparatuses 30 having output a login signal. When an interactive switching signal is received from any one of the host apparatuses 30 having output a login signal, the communication managing portion 101 blocks input of the examiner audio information and the examiner image-capture information to the ophthalmic apparatus 10.

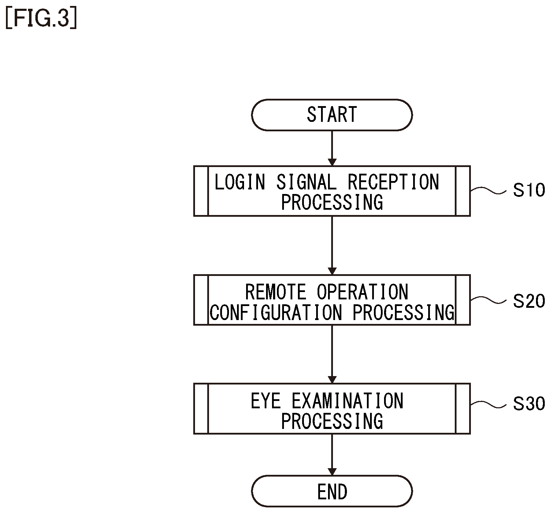

A flow of processing of an eye examination in the ophthalmic system 1 according to the first embodiment is described below. As shown in FIG. 3, in an eye examination in the ophthalmic system 1 according to the first embodiment, first, the communication managing portion 101 executes login signal reception processing (step S10). Next, the communication managing portion 101 executes remote operation configuration processing (step S20). This enables the operation host apparatus 30α to remotely operate the ophthalmic apparatus 10. In addition, eye examination processing (step S30) is executed by remote operation of the ophthalmic apparatus 10 by the operation host apparatus 30α.

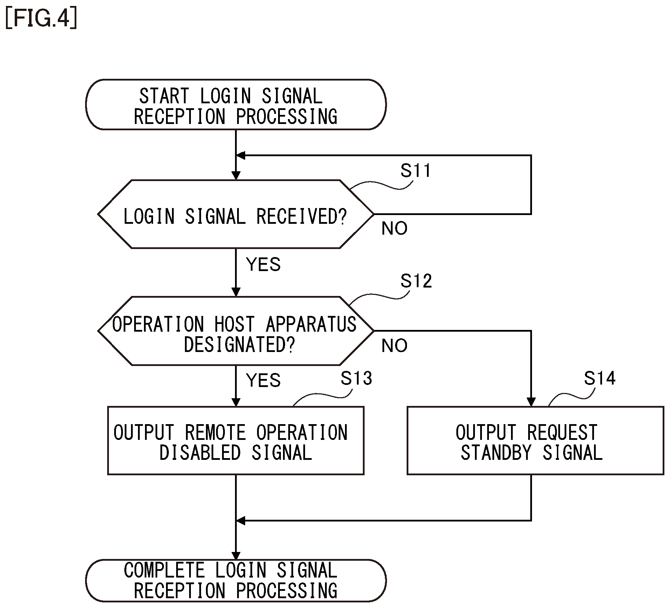

Hereinafter, FIG. 4 illustrates a flow of the login signal reception processing in step S10. The login signal reception processing is repetitively executed from the time that the communication managing portion 101 starts up until it shuts down.

In step S11, following the start of the login signal reception processing, the communication managing portion 101 determines whether or not a login signal has been received from any of the plurality of host apparatuses 30. When a determination of YES (login signal has been received) is made, the communication managing portion 101 proceeds to step S12, but when a determination of NO (login signal has not been received) is made, the communication managing portion 101 repeats step S11. The login signal is output when an output instruction thereof is input to the main body control portion 36 as a result of the examiner operating the operating portion 31. In the host apparatus 30 having output the login signal, the main body control portion 36 activates each of the examiner monitor 32, the examiner speaker 33, the examiner microphone 34, and the examiner camera 35.

In step S12, following the determination that a login signal has been received in step S11, the communication managing portion 101 determines whether or not the operation host apparatus 30α has already been designated. When a determination of YES (operation host apparatus 30α has been designated) is made, the communication managing portion 101 proceeds to step S13, but when a determination of NO (operation host apparatus 30α has not been designated) is made, the communication managing portion 101 proceeds to step S14.

In step S13, following the determination that the operation host apparatus 30α has been designated in step S12, the communication managing portion 101 outputs a remote operation disabled signal to any other host apparatus 30 having output the login signal and proceeds to complete the login signal reception processing. In the host apparatus 30 having received the "remote operation disabled signal", the main body control portion 36 may cause, for example, the examiner monitor 32 to display a message to the effect that the ophthalmic apparatus 10 cannot be operated.

In step S14, following the determination that the operation host apparatus 30α has not been designated in step S12, the communication managing portion 101 outputs a request standby signal to the host apparatus 30 having output the login signal and proceeds to complete the login signal reception processing. In the host apparatus 30 having received the "request standby signal", the main body control portion 36 may, for example, cause the examiner monitor 32 to display a message to the effect that the host apparatus 30 is standing by to receive a request signal from the ophthalmic apparatus 10.

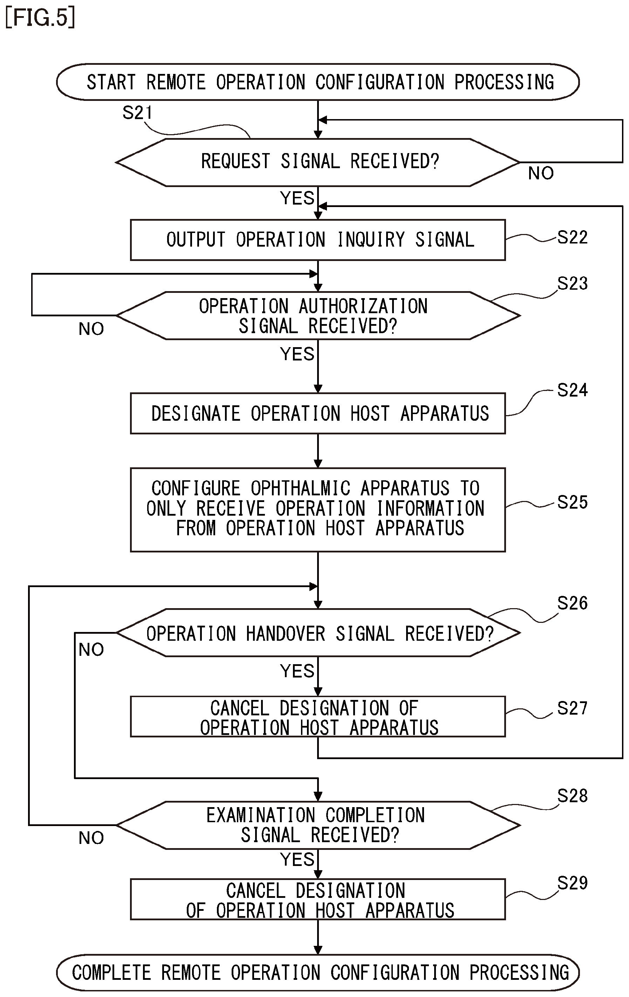

Hereinafter, FIG. 5 illustrates a flow of the remote operation configuration processing in step S20. The remote operation configuration processing repetitively executes from the time the communication managing portion 101 starts up until it shuts down.

In step S21, following the start of the remote operation configuration processing, the communication managing portion 101 determines whether or not a request signal has been received from the ophthalmic apparatus 10. When a determination of YES (request signal has been received) is made, the communication managing portion 101 proceeds to step S22, but when a determination of NO (request signal has not been received) is made, the communication managing portion 101 repeats step S21. In the ophthalmic apparatus 10 having output the request signal, the apparatus control portion 19 may, for example, cause the subject monitor 15 to display a message to the effect that the ophthalmic apparatus 10 is standing by for remote operation by the host apparatus 30.

In step S22, following the determination that a request signal has been received in step S21 or cancellation of designation of the operation host apparatus 30α in step S27, the communication managing portion 101 outputs an operation inquiry signal to all host apparatuses 30 having received a request standby signal (host apparatuses 30 having output a login signal) and proceeds to step S23. In the host apparatus 30 having received the operation inquiry signal, the main body control portion 36 may, for example, cause the examiner monitor 32 to display a message to the effect that the host apparatus 30 has received the operation inquiry signal.

In step S23, following the output of the operation inquiry signal in step S22, the communication managing portion 101 determines whether or not an operation authorization signal has been received from any of the host apparatuses 30 having received the operation inquiry signal. When a determination of YES (operation authorization signal has been received) is made, the communication managing portion 101 proceeds to step S24, but when a determination of NO (operation authorization signal has not been received) is made, the communication managing portion 101 repeats step S23. The operation authorization signal is output when an output instruction thereof is received as a result of an operation of the operating portion 31 by the examiner having recognized that the operation inquiry signal has been received. When operation authorization signals are simultaneously received from a plurality of host apparatuses 30, the communication managing portion 101 receives the operation authorization signal from any one host apparatus 30 based on predetermined criteria.

In step S24, following the determination that an operation authorization signal has been received in step S23, the communication managing portion 101 designates the operation host apparatus 30α, which is the host apparatus authorized to remotely operate the ophthalmic apparatus 10, and proceeds to step S25. At this point, the communication managing portion 101 designates the host apparatus 30 having output the operation authorization signal (the host apparatus 30 having received the operation authorization signal) as the "operation host apparatus 30α."

In step S25, following the designation of the operation host apparatus 30α in step S24, the communication managing portion 101 recognizes that the operation host apparatus 30α is capable of performing an eye examination and configures the ophthalmic apparatus 10 to only receive operation information output from the operation host apparatus 30α, and proceeds to step S26. This enables the operation host apparatus 30α to remotely operate the ophthalmic apparatus 10. In addition, operation information from the host apparatuses 30 other than the operation host apparatus 30α is prevented from being input to the ophthalmic apparatus 10.

In step S26, following the input configuration in step S25, the communication managing portion 101 determines whether or not an operation handover signal has been received from the operation host apparatus 30α. When a determination of YES (operation handover signal has been received) is made, the communication managing portion 101 proceeds to step S27, but when a determination of NO (operation handover signal has not been received) is made, the communication managing portion 101 proceeds to step S28.

In step S27, following the determination that an operation handover signal has been received in step S26, the communication managing portion 101 cancels the designation of the operation host apparatus 30α and returns to step S22.

In step S28, following the determination that an operation handover signal has not been received in step S26, the communication managing portion 101 determines whether or not an examination completion signal has been received from the operation host apparatus 30α. When a determination of YES (examination completion signal has been received) is made, the communication managing portion 101 proceeds to step S29, but when a determination of NO (examination completion signal has not been received) is made, the communication managing portion 101 returns to step S26.

In step S29, following the determination that an examination completion signal has been received in step S28, the communication managing portion 101 cancels the designation of the operation host apparatus 30α and proceeds to complete the remote operation configuration processing.

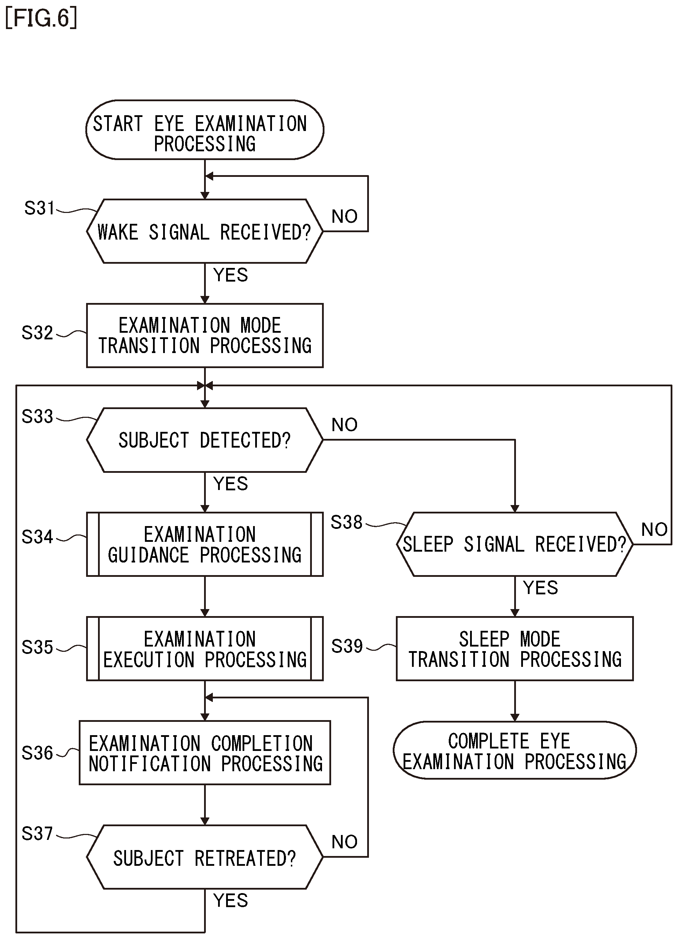

Hereinafter, FIG. 6 illustrates a flow of the eye examination processing in the ophthalmic apparatus 10 by remote operation by the operation host apparatus 30α in step S30. The ophthalmic apparatus 10 remains in a sleep mode until remote operation starts. Accordingly, the ophthalmic apparatus 10 starts the eye examination processing from the sleep mode.

In step S31, following the start of the eye examination processing, the apparatus control portion 19 determines whether or not a wake signal from the operation host apparatus 30α has been received. When a determination of YES (wake signal has been received) is made, the apparatus control portion 19 proceeds to step S32, but when a determination of NO (wake signal has not been received) is made, the apparatus control portion 19 repeats step S31. At this point, the ophthalmic apparatus 10 enters a sleep mode after outputting a request signal. In the operation host apparatus 30α, once a wake signal is output from the main body control portion 36 in response to an operation input by the examiner to the operating portion 31, the apparatus control portion 19 receives the wake signal.

In step S32, following the determination that a wake signal has been received in step S31, the apparatus control portion 19 transitions to an examination mode and proceeds to step S33. In the examination mode transition processing, first, the apparatus control portion 19 activates each of the chin rest 12e, the drive mechanism 13, the measurement head 14, the subject monitor 15, the subject speakers 16, the subject microphone 17, and the subject camera 18. Next, the apparatus control portion 19 transitions the ophthalmic apparatus 10 from sleep mode to examination mode. After transitioning the ophthalmic apparatus 10 from sleep mode to examination mode, the apparatus control portion 19 may activate components such as the chin rest 12e that were previously inactive.

In step S33, following the examination mode transition processing in step S32, a determination of a retreat detection in step S37, or a determination of non-reception of a sleep signal in step S38, the apparatus control portion 19 determines whether or not the subject has been detected. When a determination of YES is made, the apparatus control portion 19 proceeds to step S34, but when a determination of NO is made, the apparatus control portion 19 proceeds to step S38. At this point, the presence of the subject is determined based on subject image-capture information. Specifically, first, subject image-capture information obtained by the subject camera 18 is output from the ophthalmic apparatus 10 to the operation host apparatus 30α. The operation host apparatus 30α causes the subject image-capture information to be displayed on the examiner monitor 32. Next, the examiner operating the operation host apparatus 30α visually checks the display on the examiner monitor 32 to confirm whether or not the subject is present.

Here, when a subject is present, the condition includes not only cases in which the entire body of the subject is captured but also cases in which only a portion of the subject, such as the head, is captured. The examiner inputs a recognition result of the presence of the subject (presence detection information or presence non-detection information) to the main body control portion 36 by inputting an operation to the operating portion 31. The main body control portion 36 outputs the presence detection information or the presence non-detection information as operation information to the ophthalmic apparatus 10. In the ophthalmic apparatus 10, the apparatus control portion 19 determines that the presence has been detected (YES) when the presence detection information is received, and the apparatus control portion 19 determines that the presence has not been detected (NO) when the presence non-detection information is received. The term "presence detection information" refers to information indicating that the examiner has recognized the presence of the subject and has thus detected the presence of the subject (presence detection). The term "presence non-detection information" refers to information indicating that the examiner has not recognized the presence of the subject and has thus not detected the presence of the subject (presence non-detection).

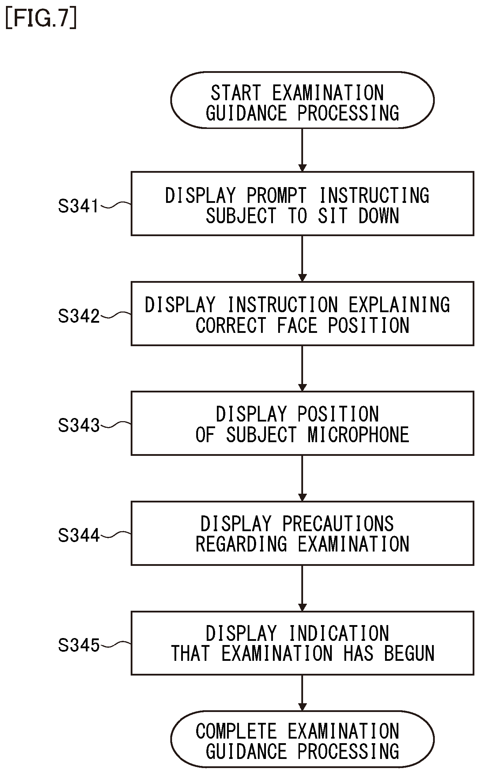

In step S34, following the determination of presence detection of the subject in step S33, the apparatus control portion 19 executes examination guidance processing of guiding the subject to the ophthalmic apparatus 10 and proceeds to step S35. The examination guidance processing is described later with reference to the flow chart in FIG. 7.

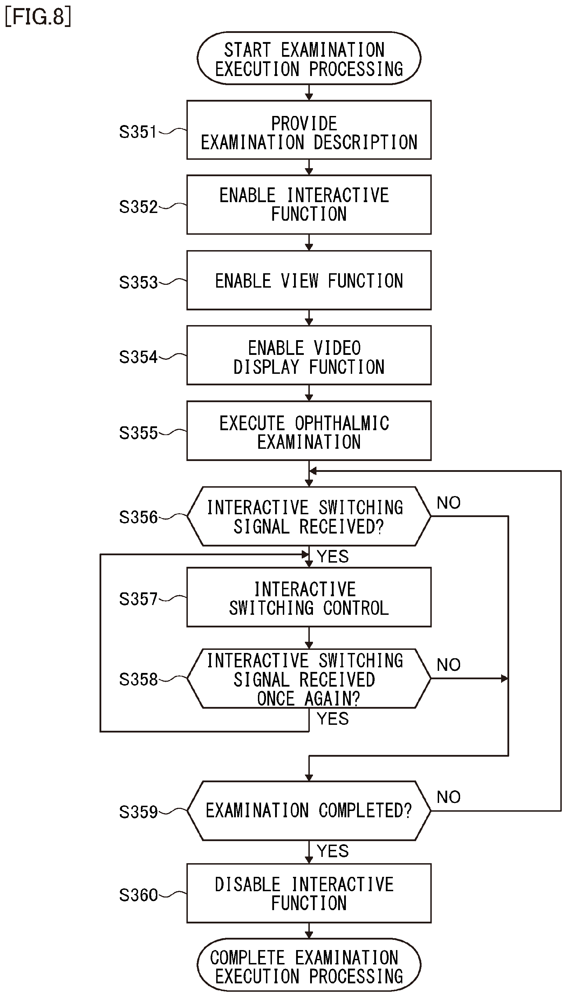

In step S35, following the examination guidance processing in step S34, the apparatus control portion 19 performs examination execution processing of executing eye examination using the ophthalmic apparatus 10 and proceeds to step S36. The examination execution processing is described later with reference to the flow chart in FIG. 8.

In step S36, following the examination execution processing in step S35 or the determination of a retreat non-detection in step S37, the apparatus control portion 19 executes examination completion notification processing and proceeds to step S37. The examination completion notification processing is processing of notifying the subject that the examination has been completed. In the present processing, when the apparatus control portion 19 proceeds to step S36, the subject speakers 16 output automated audio information in response to automatically receiving the audio information from the apparatus control portion 19. Alternatively, when the subject monitor 15 receives completion notification display information notifying completion from the apparatus control portion 19, the subject monitor 15 may display the information. Examples of the automated audio information and the completion notification display information include audio information and text information such as "Please move your face out of the frame. All examinations have been completed. Thank you for your cooperation." At this point, when the apparatus control portion 19 returns to step S36 following a determination of retreat non-detection in step S37, the subject will be prompted to retreat once again.

In step S37, following the examination completion notification processing in step S36, the apparatus control portion 19 determines whether or not the subject has retreated. When a determination of YES is made, the apparatus control portion 19 returns to step S33, but when a determination of NO is made, the apparatus control portion 19 returns to step S36. At this point, whether or not the subject has retreated is determined based on subject image-capture information. Specifically, first, subject image-capture information obtained by the subject camera 18 is output from the apparatus control portion 19 to the main body control portion 36 of the operation host apparatus 30α. The main body control portion 36 of the operation host apparatus 30α causes the subject image-capture information to be displayed on the examiner monitor 32 of the operation host apparatus 30α. Next, the examiner operating the operation host apparatus 30α visually checks the display on the examiner monitor 32 to confirm whether or not the subject has retreated.

The subject is determined to have retreated when the subject's entire body is not visible and not when a part of the subject such as the head is visible. The examiner inputs a recognition result of a retreat by the subject (retreat detection information or retreat non-detection information) to the main body control portion 36 by an operation of the operating portion 31. The term "retreat detection information" refers to information indicating that the examiner has not recognized the presence of the subject but has recognized a retreat by the subject and has thus detected a retreat by the subject (retreat detection). The term "retreat non-detection information" refers to information indicating that the examiner has recognized the presence of the subject but has not recognized a retreat by the subject and has thus not detected a retreat by the subject (retreat non-detection). The main body control portion 36 outputs the retreat detection information or the retreat non-detection information as operation information to the apparatus control portion 19. When the retreat detection information is received, the apparatus control portion 19 determines that a retreat has been detected (YES), but when the retreat non-detection information is received, the apparatus control portion 19 determines that the retreat has not been detected (NO).

In step S38, following the determination of presence non-detection of the subject in step S33, the apparatus control portion 19 determines whether or not a sleep signal from the operation host apparatus 30α has been received. When a determination of YES (sleep signal has been received) is made, the apparatus control portion 19 proceeds to step S39, but when a determination of NO (sleep signal has not been received) is made, the apparatus control portion 19 returns to step S33. At this point, when the ophthalmic apparatus 10 is in the examination mode, the apparatus control portion 19 determines that a sleep signal is received in response to output of the sleep signal from the main body control portion 36 of the operation host apparatus 30α.

In step S39, following the determination that a sleep signal has been received in step S38, the apparatus control portion 19 executes sleep mode transition processing and proceeds to complete the eye examination processing. In the sleep mode transition processing, first, the apparatus control portion 19 deactivates each of the chin rest 12e, the drive mechanism 13, the measurement head 14, the subject monitor 15, the subject speakers 16, the subject microphone 17, and the subject camera 18. Next, the apparatus control portion 19 transitions the ophthalmic apparatus 10 from examination mode to sleep mode. Once a transition of the ophthalmic apparatus 10 to sleep mode is confirmed, the operation host apparatus 30α outputs an examination completion signal. The examiner operating the operation host apparatus 30α confirms the transition to sleep mode by checking mode information of the ophthalmic apparatus 10.

Hereinafter, the flow of the examination guidance processing in step S34 is described with reference to FIG. 7.

In step S341, following the start of the examination guidance processing, a prompt instructing the subject to sit down is displayed on the subject monitor 15 and the processing proceeds to step S342.

In step S342, following the display in step S341, an instruction explaining a correct position for the subject's face is displayed on the subject monitor 15 and the processing proceeds to step S343.

In step S343, following the display in step S342, the position of the subject microphone 17 is displayed on the subject monitor 15 and the processing proceeds to step S344.

In step S344, following the display in step S343, precautions regarding the examination by the ophthalmic apparatus 10 are displayed on the subject monitor 15 and the processing proceeds to step S345.

In step S345, following the display in step S344, the subject monitor 15 displays an indication that the examination by the ophthalmic apparatus 10 has begun and the processing proceeds to complete the examination guidance processing.

Hereinafter, FIG. 8 illustrates a flow of the examination execution processing in step S35.

In step S351, following the start of the examination execution processing, the apparatus control portion 19 provides an examination explanation and proceeds to step S352. Providing the examination explanation involves explaining that the examination is about to begin and explaining that the subject can communicate with the examiner by speaking into the subject microphone 17 if they have any questions or concerns. The examination explanation is provided when the processing proceeds to step S351, and automated audio information having been automatically input from the apparatus control portion 19 to the subject speakers 16 causes the automated audio information to be output therefrom. Examples of the audio information of the examination explanation include statements such as "A refraction examination will now be performed. If you have any questions or concerns, please speak into the microphone to communicate with us."

In step S352, following the execution of the examination explanation in step S351, an interactive function is enabled and the processing proceeds to step S353. The term "interactive function" refers to a function that enables communication between a subject undergoing an eye examination by the ophthalmic apparatus 10 and the examiners operating any of the host apparatuses 30 having output a login signal including the operation host apparatus 30α.

In other words, when enabling the interactive function, the communication managing portion 101 allows the subject audio information captured by the subject microphone 17 and output from the ophthalmic apparatus 10 to be input to all host apparatuses 30 having output a login signal. Each of the host apparatuses 30 outputs subject audio information through an examiner speaker 33 upon receiving the subject audio information. In addition, the communication managing portion 101 allows examiner audio information acquired by the examiner microphone 34 in all host apparatuses 30 having output a login signal to be input to the ophthalmic apparatus 10. The ophthalmic apparatus 10 outputs examiner audio information through the subject speakers 16 upon receiving the examiner audio information.

When the interactive function is ON, according to the first embodiment, the system allows examiners controlling the host apparatuses 30 having output a login signal to communicate with one another. In other words, the communication managing portion 101 allows the examiner audio information output from one host apparatus 30 to be input to the other host apparatuses 30 having output a login signal. Each of the host apparatuses 30 outputs examiner audio information through an examiner speaker 33 upon receiving the examiner audio information.

In step S353, following the enabling of the interactive function in step S352, a view function is enabled and the processing proceeds to step S354. The term "view function" refers to a function that enables examination result information obtained by the ophthalmic apparatus 10 to be viewed on all host apparatuses 30 having output a login signal including the operation host apparatus 30α.

In other words, when enabling the view function, the communication managing portion 101 allows the examination result information output from the ophthalmic apparatus 10 to be input to all host apparatuses 30 having output a login signal. Upon receiving the examination result information, each of the host apparatuses 30 displays the examination result information on the examiner monitor 32.

In step S354, following the enabling of the view function in step S353, a video display function is enabled and the processing proceeds to step S355. The term "video display function" refers to a function that allows the subject image-capture information to be viewable by the examiners and the examiner image-capture information to be viewable by the subject.

In other words, when enabling the video display function, the communication managing portion 101 allows the subject image-capture information captured by the subject camera 18 to be input to all host apparatuses 30 having output a login signal. Upon receiving the subject image-capture information, each of the host apparatuses 30 displays the subject image-capture information on the examiner monitor 32. In addition, the communication managing portion 101 allows the examiner image-capture information captured by the examiner camera 35 in all host apparatuses 30 having output a login signal to be input to the ophthalmic apparatus 10. Upon receiving the examiner image-capture information, the ophthalmic apparatus 10 displays the examiner image-capture information on the subject monitor 15.

In step S355, following the enabling of the video display function in step S354, an eye examination is actually executed and the processing proceeds to step S356. The eye examination is performed by inputting the operation information output from the operation host apparatus 30α to the ophthalmic apparatus 10. Specifically, in the ophthalmic apparatus 10, the drive mechanism 13 is subjected to drive control based on the operation information to adjust a position of the chin rest 12e in the Y-direction and roughly align the optical system 14A in the X-, Y-, and Z-directions relative to the eye. In addition, the ophthalmic apparatus 10 controls the execution of examinations necessary for an eye examination based on the operation information.

In step S356, following the execution of the eye examination in step S355 or a determination of eye examination non-completion in step S359, the communication managing portion 101 determines whether or not an interactive switching signal has been received. When the communication managing portion 101 makes a determination of YES (interactive switching signal has been received), the processing proceeds to step S357, but when a determination of NO (interactive switching signal has not been received) is made, the processing proceeds to step S359. At this point, when an interactive switching signal is output from the main body control portion 36 in response to an operation of the operating portion 31 by an examiner, the interactive switching signal is input to the communication managing portion 101. The interactive switching signal can be input from all of the host apparatuses 30 having output a login signal.

In step S357, following a determination that an interactive switching signal has been received in step S356 or an interactive switching signal is received once again in step S358, interactive switching control is performed and the processing proceeds to step S358. In "interactive switching control", when communication between the subject and the examiners is enabled, the communication between the subject and the examiners is interrupted and communication only among the examiners controlling the host apparatuses 30 having output a login signal is enabled. Alternatively, when communication only among the examiners controlling the host apparatuses 30 having output a login signal is enabled, the communication among the examiners is interrupted and communication between the subject and the examiners is enabled.

In other words, in interactive switching control, when communication between the subject and the examiners is enabled, the communication managing portion 101 prevents at least the examiner audio information output from each of the host apparatuses 30 having output a login signal from being input to the ophthalmic apparatus 10. Accordingly, communication between the subject and the examiners is disabled. The examiner audio information output from the host apparatus 30 can be input to the main body control portion 36 of the other host apparatuses 30 in step S352. Consequently, when each of the host apparatuses 30 receives examiner audio information, outputting the examiner audio information from the examiner speaker 33 enables communication only between examiners. At this point, input of subject audio information to each of the host apparatuses 30 may be either enabled or restricted.

When communication is enabled only among examiners, the communication managing portion 101 allows the examiner audio information output from each of the host apparatuses 30 having output a login signal to be input to the ophthalmic apparatus 10. Upon receiving the examiner audio information, the ophthalmic apparatus 10 causes the examiner audio information to be output from the subject speakers 16. Accordingly, communication between the subject and the examiners is once again enabled. A state where the examiner audio information can be input to other host apparatuses 30 is maintained regardless of whether or not examiner audio information can be input to the ophthalmic apparatus 10. When input of subject audio information to the host apparatuses 30 is restricted, the communication managing portion 101 allows subject audio information to be input to the host apparatuses 30.

In step S358, following the interactive switching control in step S357, the communication managing portion 101 determines whether or not an interactive switching signal has been received once again. When the communication managing portion 101 makes a determination of YES (interactive switching signal has been received once again), the processing returns to step S357, but when a determination of NO (interactive switching signal has not been received once again) is made, the processing proceeds to step S359. At this point, when an interactive switching signal is output once again from the main body control portion 36 in response to an operation of the operating portion 31 by an examiner, the communication managing portion 101 receives the interactive switching signal once again. The interactive switching signal can be received once again from all of the host apparatuses 30 having output a login signal.

In step S359, following a determination that an interactive switching signal has not been received in step S356 or a determination that an interactive switching signal has not been received once again in step S358, the main body control portion 36 of the operation host apparatus 30α determines whether or not the eye examination has been completed. When a determination of YES is made, the processing proceeds to step S360, but when a determination of NO is made, the processing returns to step S356. The ophthalmic examination is completed when examinations for all preset examination items have been completed. For this reason, the main body control portion 36 determines whether or not all examination items set in advance have been completed based on the operation information and the examination result information.

In step S360, following the determination of examination completion in step S359, at least the interactive function is disabled and the processing proceeds to complete the examination execution processing. In other words, in the interactive function OFF control, the communication managing portion 101 prevents the subject audio information output from the ophthalmic apparatus 10 from being input to all host apparatuses 30 having output a login signal. The communication managing portion 101 also prevents examiner audio information output from each of the host apparatuses 30 from being input to the ophthalmic apparatus 10. The communication managing portion 101 prevents examiner audio information output from any host apparatus 30 from being input to the other host apparatuses 30. Furthermore, at this point, the communication managing portion 101 may prevent the examination result information from being viewed on each of the host apparatuses 30. The communication managing portion 101 may prevent the subject image-capture information from being input to each of the host apparatuses 30. The communication managing portion 101 may prevent the examiner image-capture information from being input to the ophthalmic apparatus 10.

The operation of the ophthalmic system 1 according to the first embodiment is described below.

The ophthalmic system 1 according to the first embodiment includes the ophthalmic apparatus 10 and the plurality of host apparatuses 30 capable of communicating with one another. The ophthalmic apparatus 10 includes the subject microphone 17 that captures audio and the subject speakers 16 that output audio. Each of the host apparatuses 30 includes the examiner microphone 34 that captures audio, the examiner speaker 33 that outputs audio, and the examiner monitor 32 that is viewable by an examiner.

When executing an eye examination, the ophthalmic system 1 according to the first embodiment enables the interactive function (step S352). Specifically, the communication managing portion 101 allows subject audio information to be input to all host apparatuses 30 having output a login signal. Each of the host apparatuses 30 outputs the subject audio information through the examiner speaker 33 upon receiving the subject audio information. The communication managing portion 101 also allows examiner audio information obtained in all host apparatuses 30 having output a login signal to be input to the ophthalmic apparatus 10. The ophthalmic apparatus 10 outputs the examiner audio information through the subject speakers 16 upon receiving the examiner audio information.

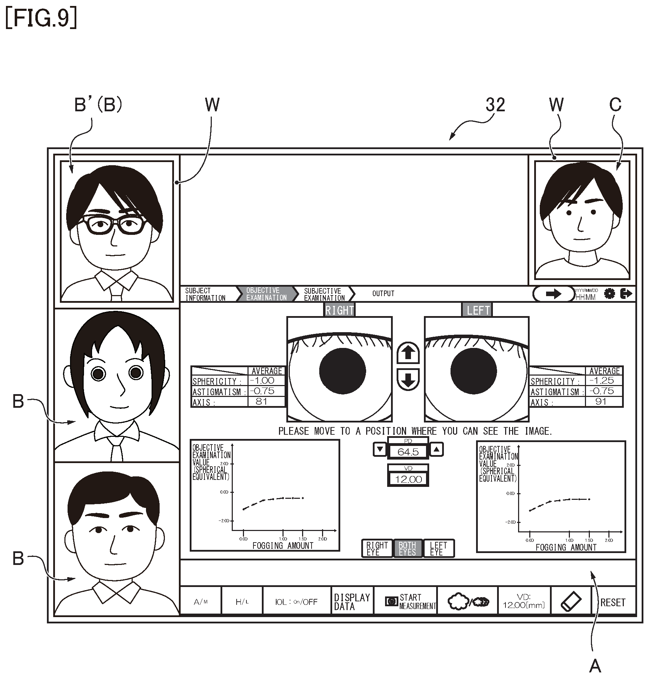

When executing an eye examination, the ophthalmic system 1 according to the first embodiment enables the view function (step S353). Specifically, the communication managing portion 101 allows examination result information to be input to all host apparatuses 30 having output a login signal. Upon receiving the examination result information, each of the host apparatuses 30 causes the examination result information to be displayed on the examiner monitor 32 (refer to FIG. 9).

In other words, in the ophthalmic system 1 according to the first embodiment, each of the host apparatuses 30 having output a login signal causes the result of the eye examination (examination result information) to be displayed on the examiner monitor 32 and audio captured by the subject microphone 17 to be output from the examiner speaker 33. The ophthalmic apparatus 10 outputs audio captured by the examiner microphone 34 through the subject speakers 16.

Accordingly, the examiner operating each of the host apparatuses 30 having output a login signal can confirm the results of the eye examination by viewing the examiner monitor 32. Each examiner and the subject can communicate with one another via the subject microphone 17, the examiner speaker 33, the examiner microphone 34, and the subject speakers 16. As a result, the plurality of host apparatuses 30 can share the examination result information. A plurality of examiners can confirm the result of the eye examination and the condition of the subject, and the subject can ask the plurality of examiners questions. Therefore, even if a login signal is not output from any of the host apparatuses 30 due to, for example, the absence of an examiner or another reason, the result of the eye examination can be confirmed by the examiners operating the other host apparatuses 30, allowing the eye examination to proceed smoothly. The ophthalmic system 1 according to the first embodiment enables an eye examination using the ophthalmic apparatus 10 to be performed under the supervision of at least one examiner.

In the ophthalmic system 1 according to the first embodiment, the plurality of host apparatuses 30 can communicate with one another. In the interactive function ON control according to the first embodiment, communication among examiners operating host apparatuses 30 that have output a login signal is enabled (step S352). In other words, the communication managing portion 101 allows the examiner audio information obtained by one host apparatus 30 to be input to the other host apparatuses 30. Each of the host apparatuses 30 outputs the examiner audio information through the examiner speaker 33 upon receiving the examiner audio information. In other words, each of the host apparatuses 30 causes audio captured by the examiner microphone 34 to be output from the examiner speaker 33 included in other host apparatuses 30 and causes audio captured by the examiner microphone 34 included in the other host apparatuses 30 to be output from the examiner speaker 33.

Accordingly, the ophthalmic system 1 according to the first embodiment enables the plurality of examiners operating the host apparatuses 30 having output a login signal to communicate with one another via the examiner microphone 34 and the examiner speaker 33. The plurality of examiners can exchange opinions and share information regarding the results of eye examinations and other matters.

In the ophthalmic system 1 according to the first embodiment, when an interactive switching signal is input while communication between the subject and the examiners is enabled, the communication managing portion 101 prevents the ophthalmic apparatus 10 from receiving the examiner audio information obtained by each of the host apparatuses 30 having output a login signal (step S357).

In other words, in the ophthalmic system 1 according to the first embodiment, the subject speakers 16 can restrict the output of audio captured by the examiner microphone 34 when the audio is output from the subject speakers 16. Accordingly, when the plurality of examiners communicate with one another, the ophthalmic system 1 according to the first embodiment can prevent the subject from overhearing their conversations, thereby preventing unnecessary leakage or disclosure of information.

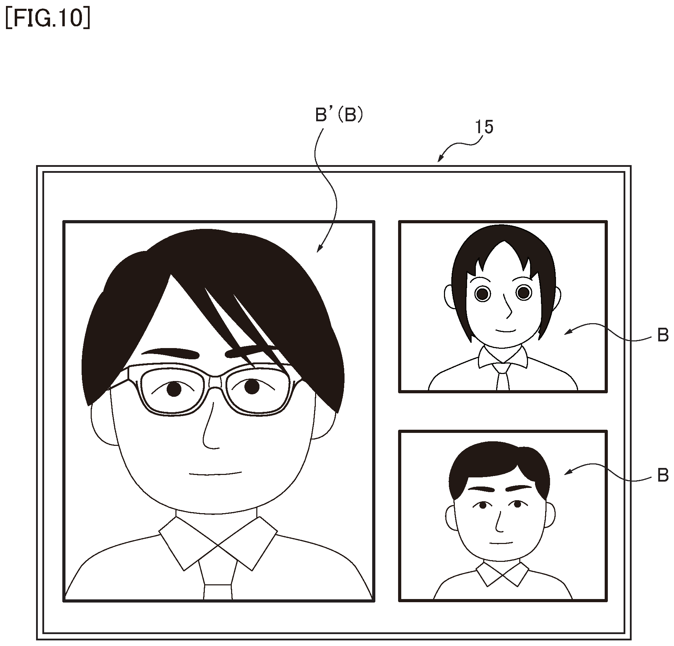

In the ophthalmic system 1 according to the first embodiment, each of the plurality of host apparatuses 30 includes the examiner camera 35 capable of capturing an examiner and the ophthalmic apparatus 10 includes the subject monitor 15 that is viewable by the subject and the subject camera 18 capable of capturing the subject. When executing an eye examination, the video display function is enabled (step S354). Specifically, the communication managing portion 101 enables the main body control portion 36 of all host apparatuses 30 having output a login signal to receive subject image-capture information. Upon receiving the subject image-capture information, each of the main body control portions 36 causes the subject image-capture information to be displayed on the examiner monitor 32 (refer to FIG. 9). The communication managing portion 101 also enables the apparatus control portion 19 of the ophthalmic apparatus 10 to receive examiner image-capture information. Upon receiving the examiner image-capture information, the apparatus control portion 19 causes the examiner image-capture information to be displayed on the subject monitor 15 (refer to FIG. 10).

In other words, in the ophthalmic system 1 according to the first embodiment, each of the host apparatuses 30 having output a login signal causes the subject image-capture information to be displayed on the examiner monitor 32 and the ophthalmic apparatus 10 causes the examiner image-capture information to be displayed on the subject monitor 15.

Accordingly, the examiner operating each of the host apparatuses 30 having output a login signal can visually confirm the condition of the subject by viewing the examiner monitor 32. The subject can visually confirm the examiner with whom they are communicating by viewing the subject monitor 15. The ophthalmic system 1 according to the first embodiment enables each examiner and the subject to communicate while looking at each other's faces.

FIG. 9 illustrates an example of a display screen on the examiner monitor 32 of all host apparatuses 30 having output a login signal, including the operation host apparatus 30α during an eye examination. FIG. 10 illustrates an example of a display screen on the subject monitor 15 during an eye examination.

As shown in FIG. 9, the examiner monitor 32 displays examination result information A representing information including a result of an eye examination, a plurality of pieces of examiner image-capture information B including facial images of examiners operating the host apparatuses 30, and subject image-capture information C including a facial image of the subject. Examiner image-capture information B' output from the operation host apparatus 30α may be displayed, for example, enclosed in a frame W. Accordingly, the host apparatuses 30 enable the examiners to readily distinguish the examiner image-capture information B' output from the operation host apparatus 30α. The subject image-capture information C may also be displayed, for example, enclosed in the frame W. Accordingly, the host apparatuses 30 enable the examiners to readily distinguish the subject image-capture information C.

Information displayed enclosed in the frame W is not limited to the examiner image-capture information B' output from the operation host apparatus 30α. For example, the image of the examiner speaking or, in other words, the examiner image-capture information B in the host apparatus 30 having output the examiner audio information being output from the examiner speaker 33 may be displayed enclosed in the frame W.

As shown in FIG. 9, the examination result information A is displayed on the examiner monitor 32 in all of the host apparatuses 30 having output a login signal. Accordingly, the examiner operating each of the host apparatuses 30 can view and comprehend the results of the eye examination. The examination results can be shared among the plurality of host apparatuses 30, facilitating exchange of opinions and sharing of information among the examiners.

As shown in FIG. 10, the subject monitor 15 displays the plurality of pieces of examiner image-capture information B including facial images of the examiners operating the host apparatuses 30. At this point, a region where the examiner image-capture information B' output from the operation host apparatus 30α is displayed may be set to be the largest. Accordingly, the ophthalmic apparatus 10 enables the subject to readily distinguish the examiner image-capture information B' output from the operation host apparatus 30α. Information for which the display region is set the largest is not limited to the examiner image-capture information B' output from the operation host apparatus 30α. For example, the region displaying the image of the examiner speaking or, in other words, the examiner image-capture information B in the host apparatus 30 having output the examiner audio information being output from the subject speakers 16 may be set to be the largest.

When the output of examiner audio information from the subject speakers 16 is restricted, the communication managing portion 101 may restrict the input of examiner image-capture information to the ophthalmic apparatus 10. In this case, the ophthalmic apparatus 10 does not display examiner image-capture information on the subject monitor 15, allowing the examiners to communicate with one another without the subject's knowledge.

In the ophthalmic system 1 according to the first embodiment, during the remote operation configuration processing, when a request signal is input from the ophthalmic apparatus 10 and an operation authorization signal is input from the host apparatuses 30 having output a login signal, the communication managing portion 101 designates the operation host apparatus 30α to remotely operate the ophthalmic apparatus 10 (steps S21 to S24). The communication managing portion 101 configures the ophthalmic apparatus 10 to only receive operation information output from the operation host apparatus 30α (step S25).

In other words, in the ophthalmic system 1 according to the first embodiment, the ophthalmic apparatus 10 is remotely operated based on operation information output from any one of the plurality of host apparatuses 30 (operation host apparatus 30α) and, during the remote operation, the ophthalmic apparatus 10 is prevented from receiving operation information output from the other host apparatuses 30.

Accordingly, the ophthalmic system 1 according to the first embodiment restricts the output of operation information to be received by the ophthalmic apparatus 10 from the plurality of host apparatuses 30, thereby enabling appropriate remote operation of the ophthalmic apparatus 10.

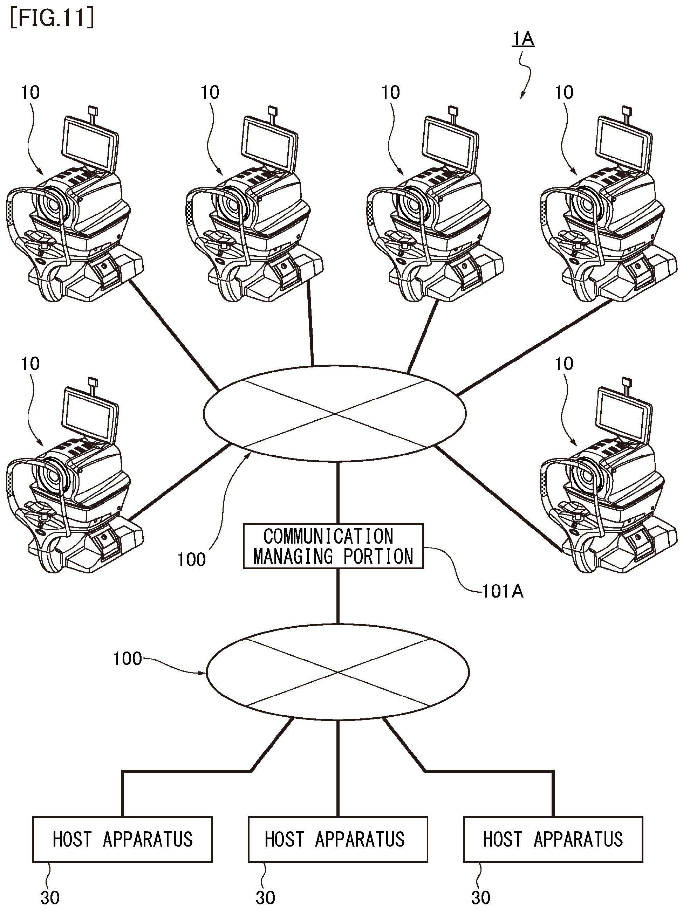

(Second Embodiment) As shown in FIG. 11, an ophthalmic system 1A according to the second embodiment includes a plurality of ophthalmic apparatuses 10, which include six ophthalmic devices in this example, a plurality of host apparatuses 30, which include three host apparatuses in this example, a communication network 100, and a communication managing portion 101A. In the ophthalmic system 1A according to the second embodiment, the ophthalmic apparatuses 10 outnumber the host apparatuses 30.

Similar to the first embodiment, each of the ophthalmic apparatuses 10 according to the second embodiment is remotely operated based on a control signal transmitted from any one of the host apparatuses 30. Furthermore, similar to the first embodiment, the results of an eye examination by the ophthalmic apparatus 10 are displayed so as to be viewable on all of the plurality of host apparatuses 30.

The communication managing portion 101A according to the second embodiment is configured to enable one of the plurality of host apparatuses 30 to perform remote operation in an order of request signals output from the plurality of ophthalmic apparatuses 10.

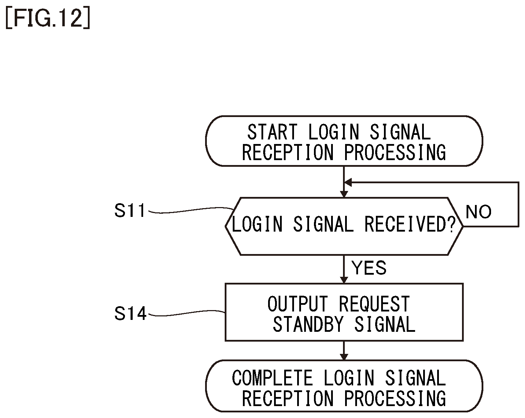

FIG. 12 illustrates a flow of login signal reception processing in the ophthalmic system 1A according to the second embodiment.

In step S11, following the start of the login signal reception processing, the communication managing portion 101A determines whether or not a login signal has been received from the host apparatus 30. When a determination of YES (login signal has been received) is made, the communication managing portion 101A proceeds to step S14, but when a determination of NO (login signal has not been received) is made, the communication managing portion 101A repeats step S11.

In step S14, following the determination that a login signal has been received in step S11, the communication managing portion 101A outputs a request standby signal to the host apparatus 30 having output the login signal and proceeds to complete the login signal reception processing. In the host apparatus 30 having received the "request standby signal", the main body control portion 36 may, for example, cause the examiner monitor 32 to display a message to the effect that the host apparatus 30 is standing by to receive a request signal from the ophthalmic apparatus 10.

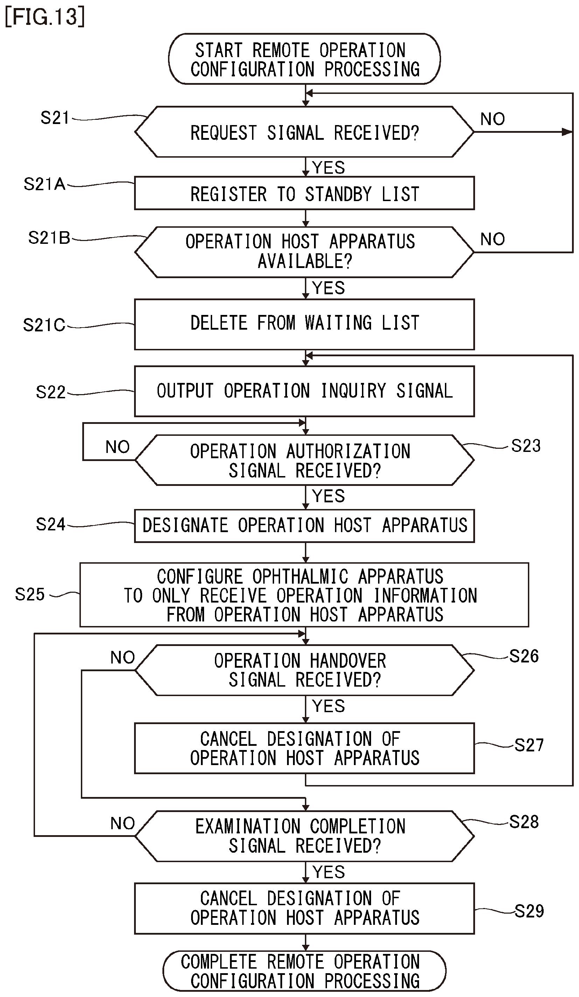

Hereinafter, FIG. 13 illustrates a flow of the remote operation configuration processing according to the second embodiment. Since the respective flows of the eye examination processing, the examination guidance processing, and the examination execution processing in the second embodiment are the same as in the first embodiment, detailed descriptions will not be repeated.

In step S21, following the start of the remote operation configuration processing or the determination in step S21B that the operation host apparatus 30α is not available, the communication managing portion 101A determines whether or not a request signal has been received from the ophthalmic apparatus 10. When a determination of YES (request signal has been received) is made, the communication managing portion 101A proceeds to step S21A, but when a determination of NO (request signal has not been received) is made, the communication managing portion 101A repeats step S21.

In step S21A, following the determination that a request signal has been received in step S21, the communication managing portion 101A registers information of the ophthalmic apparatus 10 having output the request signal to a standby list stored in a memory (not illustrated), updates the standby list, and proceeds to step S21B. Accordingly, information of the ophthalmic apparatus 10 having output the request signal is sequentially registered to the standby list. In the ophthalmic apparatus 10 registered to the standby list, for example, the apparatus control portion 19 may cause the subject monitor 15 to display a message to the effect that the ophthalmic apparatus 10 is standing by for remote operation by the host apparatus 30.

In step S21B, following the registration to the standby list in step S21A, the communication managing portion 101A determines whether or not the operation host apparatus 30α is available. When a determination of YES (the operation host apparatus 30α is available) is made, the communication managing portion 101A proceeds to step S21C, but when a determination of NO (the operation host apparatus 30α is not available) is made, the communication managing portion 101A returns to step S21. At this point, when all host apparatuses 30 having received a request standby signal (host apparatuses 30 having output a login signal) are designated as the operation host apparatus 30α, a determination that the operation host apparatus 30α is not available is made. On the other hand, when at least one of the host apparatuses 30 having received a request standby signal (host apparatuses 30 having output a login signal) is not designated as the operation host apparatus 30α, a determination that the operation host apparatus 30α is available is made.

In step S21C, following the determination that the operation host apparatus 30α is available in step S21B, the communication managing portion 101A reads the standby list from the memory (not illustrated), deletes the information of the ophthalmic apparatus 10 that was registered to the standby list first, and proceeds to step S22. When the information of the ophthalmic apparatus 10 is deleted, the order of the pieces of information of the ophthalmic apparatuses 10 registered to the standby list is moved up.

In step S22, following the deletion of the information in the standby list in step S21C or cancellation of designation of the operation host apparatus 30α in step S27, the communication managing portion 101A outputs an operation inquiry signal to host apparatuses 30 not designated as the operation host apparatus 30α among the host apparatuses 30 having received a request standby signal (host apparatuses 30 having output a login signal) and proceeds to step S23. In the host apparatus 30 having received the operation inquiry signal, the main body control portion 36 may, for example, cause the examiner monitor 32 to display a message to the effect that the host apparatus 30 has received the operation inquiry signal.

The processing from step S23 to step S29 is the same as in the first embodiment. Therefore, a detailed description of these steps will not be repeated.

In step S25, the ophthalmic apparatus 10 of which information was deleted in step S21C (the ophthalmic apparatus 10 that was registered to the standby list first) is configured to only receive operation information output from the operation host apparatus 30α. This enables the operation host apparatus 30α to remotely operate the ophthalmic apparatus 10 that was registered to the standby list first. In addition, the ophthalmic apparatus 10 that was registered to the standby list first is prevented from receiving operation information from the host apparatuses 30 other than the operation host apparatus 30α.

As described above, in the ophthalmic system 1A according to the second embodiment, the communication managing portion 101A has a standby list, and when the communication managing portion 101A receives a request signal from an ophthalmic apparatus 10, information of the ophthalmic apparatus 10 having output the request signal is sequentially registered to the standby list (step S21A). The ophthalmic system 1A then determines whether or not the operation host apparatus 30α is available (step S21B).

At this point, when it is determined that the operation host apparatus 30α is available, the information of the ophthalmic apparatus 10 that was registered to the standby list first is deleted (step S21C) and, subsequently, the operation host apparatus 30α is designated from among the host apparatuses 30 having output a login signal (step S24). The ophthalmic apparatus 10 of which information was deleted in step S21C is then configured to only receive operation information output from the operation host apparatus 30α (step S25).

In this case, "the ophthalmic apparatus 10 of which information was deleted in step S21C" is the ophthalmic apparatus 10 that was registered to the standby list first. Therefore, the ophthalmic system 1A according to the second embodiment enables remote operation by the host apparatus 30 in the order of ophthalmic apparatuses 10 that output a request signal first. In addition, the designation of the operation host apparatus 30α is cancelled once the eye examination ends.

As a result, the communication managing portion 101A enables remote operation by the host apparatus 30 in the order of ophthalmic apparatuses 10 that output a request signal first after the end of the eye examination. This reduces standby time for eye examinations and increases processing speed of eye examinations.

In the ophthalmic system 1A according to the second embodiment, the plurality of ophthalmic apparatuses 10 and the plurality of host apparatuses 30 are capable of communicating with one another via the communication managing portion 101A. The communication managing portion 101A enables any one of the plurality of ophthalmic apparatuses 10 to be remotely operated based on operation information output from any one of the plurality of host apparatuses 30. The communication managing portion 101A further enables results of eye examinations conducted by a plurality of ophthalmic apparatuses 10 to be viewable by a plurality of host apparatuses 30.

Accordingly, the ophthalmic system 1A according to the second embodiment can manage the communication status between the plurality of ophthalmic apparatuses 10 and the plurality of host apparatuses 30 via the communication managing portion 101A in a centralized manner.

When enabling the interactive function in the examination execution processing, the ophthalmic system 1A according to the second embodiment enables communication only between the subject undergoing the eye examination by the ophthalmic apparatus 10 and the examiner operating the operation host apparatus 30α remotely operating the ophthalmic apparatus 10. In other words, when enabling the interactive function of the ophthalmic system 1A according to the second embodiment, the communication managing portion 101A allows the subject audio information captured by the subject microphone 17 to be only input to the operation host apparatus 30α remotely operating the ophthalmic apparatus 10. The communication managing portion 101A also allows the examiner audio information captured by the examiner microphone 34 of the operation host apparatus 30α to be only input to the ophthalmic apparatus 10 being remotely operated by the operation host apparatus 30α.

Accordingly, the subject can be prevented from overhearing audio from the host apparatuses 30 remotely controlling other ophthalmic apparatuses 10, thereby preventing unnecessary leakage or disclosure of information.

While the ophthalmic system according to the present invention has been described based on the first and second embodiments, specific configurations of the present invention are not limited to the embodiments. Various modifications and additions of design may be made without departing from the spirit and scope of the invention as set out in the accompanying claims.

The ophthalmic system 1 according to the first embodiment shows an example in which all examiners operating host apparatuses 30 having output a login signal and a subject can communicate with one another. However, for example, the examiner capable of communicating with the subject may be limited to the examiner operating the operation host apparatus 30α. In other words, even in the first embodiment, only the operation host apparatus 30α remotely operating the ophthalmic apparatus 10 may be configured to be capable of receiving the subject audio information captured by the subject microphone 17. Only the ophthalmic apparatus 10 being remotely operated by the operation host apparatus 30α may be configured to be capable of receiving the examiner audio information captured by the examiner microphone 34 of the operation host apparatus 30α.

In addition, the first and second embodiments show an example in which examiners operating a plurality of host apparatuses 30 can communicate with one another. However, the examiners need not necessarily be able to communicate with one another. For example, the examiners operating the plurality of host apparatuses 30 can exchange opinions via chat, email, phone, or other means.

In addition, the first and second embodiments show an example in which an ophthalmic apparatus 10 is remotely operated by any one of a plurality of host apparatuses 30. However, the ophthalmic apparatus 10 need not necessarily be remotely operated by the host apparatuses 30 and, for example, an operator of the ophthalmic apparatus 10, a subject, or other persons may operate the ophthalmic apparatus 10. Even in such cases, enabling results of eye examinations to be viewed using a plurality of host apparatuses 30 and enabling examiners and the subject to communicate with one another allow the examiners operating the host apparatuses 30 to issue appropriate instructions or make an appropriate diagnosis on examination results. In other words, in the ophthalmic system according to the present invention, eye examinations can be performed under the supervision of at least one or more examiners regardless of whether or not ophthalmic apparatuses 10 are remotely operated.

In addition, the first embodiment shows an example in which examiner image-capture information obtained by the examiner camera 35 is displayed on the subject monitor 15 and subject image-capture information obtained by the subject camera 18 is displayed on the examiner monitor 32. However, the information displayed on each monitor need not necessarily be image-capture information. For example, the subject monitor 15 or the examiner monitor 32 may display illustrations, still images, and other images that enable the examiners or the subject to be identified.

In addition, the first embodiment shows an example in which the subject monitor 15 of the ophthalmic apparatus 10 displays, in its entirety, examiner image-capture information output from the host apparatus 30 having output a login signal (refer to FIG. 10). However, the subject monitor 15 need only display examiner image-capture information output from at least one host apparatus 30 among the plurality of host apparatuses 30. In other words, for example, the subject monitor 15 may only display the examiner image-capture information obtained by the examiner camera 35 of the operation host apparatus 30α.

In addition, the second embodiment shows an example in which six ophthalmic apparatuses 10 and three host apparatuses 30 are connected so as to be capable of communicating with one another, with the ophthalmic apparatuses 10 outnumbering the host apparatuses 30. However, when connecting a plurality of ophthalmic apparatuses 10 and a plurality of host apparatuses 30 so as to be capable of communicating with one another, the host apparatuses 30 may outnumber the ophthalmic apparatuses 10.

Furthermore, the communication managing portion 101A need not necessarily be provided and, for example, one of the plurality of host apparatuses 30 may manage the communication status between the plurality of host apparatuses 30 and the ophthalmic apparatuses 10. In other words, for example, examination result information output from an ophthalmic apparatus 10 may be initially received by the operation host apparatus 30α and then output from the operation host apparatus 30α to the other host apparatuses 30. Even in this case, an examiner operating a host apparatus 30 other than the operation host apparatus 30α can view results of eye examinations.

The following is further disclosed regarding the descriptions of the first and second embodiments presented above.
(1) An ophthalmic system, including:
   an ophthalmic apparatus that is used in an eye examination, the ophthalmic apparatus including:
      a subject microphone that is configured to capture audio; and
      a subject speaker that is configured to output audio; and
   a plurality of host apparatuses that are operated by an examiner, the plurality of host apparatuses including:
      an examiner microphone that is configured to capture audio;
      an examiner speaker that is configured to output audio; and
      an examiner monitor that is viewable by the examiner;
   wherein
   the ophthalmic apparatus and the plurality of host apparatuses are configured to communicate with one another,
   each of the host apparatuses is configured to cause a result of the eye examination to be displayed on the examiner monitor and to cause the audio captured by the subject microphone to be output through the examiner speaker, and
   the ophthalmic apparatus is configured to cause the audio captured by the examiner microphone to be output through the subject speaker.
(2) The ophthalmic system according to (1), wherein
   each of the host apparatuses is configured to communicate with other host apparatuses, and
   each of the host apparatuses is configured to cause the audio captured by the examiner microphone to be output through the examiner speaker of the other host apparatuses and cause the audio captured by the examiner microphone of the other host apparatuses to be output through the examiner speaker of the each of the host apparatuses.
(3) The ophthalmic system according to (2), wherein the subject speaker is configured to restrict the output of the audio captured by the examiner microphone when the audio is output through the examiner speaker of the other host apparatuses.
(4) The ophthalmic system according to any one of (1) to (3), wherein
   each of the host apparatuses includes an examiner camera that is configured to capture images of the examiner,
   the ophthalmic apparatus includes a subject monitor that is viewable by a subject and a subject camera that is configured to capture images of the subject,
   each of the host apparatuses is configured to cause the examiner monitor to display image-capture information obtained by the subject camera, and
   the ophthalmic apparatus is configured to cause image-capture information obtained by the examiner camera of at least one of the plurality of host apparatuses to be displayed on the subject monitor.
(5) The ophthalmic system according to any one of (1) to (4), wherein the ophthalmic apparatus is configured to be remotely operated based on operation information output from one of the plurality of host apparatuses, and during the remote operation, to be prevented from receiving operation information output from the other host apparatuses.
(6) The ophthalmic system according to any one of (1) to (5), wherein
   the plurality of host apparatuses and a plurality of ophthalmic apparatuses are configured to communicate with one another via a communication managing portion, and
   the communication managing portion is configured to enable one of the plurality of ophthalmic apparatuses to be remotely operated based on operation information output from one of the plurality of host apparatuses and to enable results of eye examinations conducted by the plurality of ophthalmic apparatuses to be viewable by the plurality of host apparatuses.
(7) The ophthalmic system according to (6), wherein the communication managing portion is configured to enable one of the plurality of host apparatuses to perform remote operation in an order of request signals output from the plurality of ophthalmic apparatuses.

### [Cross-references to Related Applications]

This application claims priority to Japanese Patent Application No. 2023-220118, filed with the Japan Patent Office on December 26, 2023, the entire disclosure of which is incorporated herein by reference.

## Claims

1. An ophthalmic system, comprising:
an ophthalmic apparatus that is used in an eye examination, the ophthalmic apparatus comprising:
a subject microphone that is configured to capture audio; and
a subject speaker that is configured to output audio; and
a plurality of host apparatuses that are operated by an examiner, the plurality of host apparatuses comprising:
an examiner microphone that is configured to capture audio;
an examiner speaker that is configured to output audio; and
an examiner monitor that is viewable by the examiner;
wherein
the ophthalmic apparatus and the plurality of host apparatuses are configured to communicate with one another,
each of the host apparatuses is configured to cause a result of the eye examination to be displayed on the examiner monitor and to cause the audio captured by the subject microphone to be output through the examiner speaker, and
the ophthalmic apparatus is configured to cause the audio captured by the examiner microphone to be output through the subject speaker.

2. The ophthalmic system according to claim 1, wherein
each of the host apparatuses is configured to communicate with other host apparatuses, and
each of the host apparatuses is configured to cause the audio captured by the examiner microphone to be output through the examiner speaker of the other host apparatuses and cause the audio captured by the examiner microphone of the other host apparatuses to be output through the examiner speaker of the each of the host apparatuses.

3. The ophthalmic system according to claim 2, wherein the subject speaker is configured to restrict the output of the audio captured by the examiner microphone when the audio is output through the examiner speaker of the other host apparatuses.

4. The ophthalmic system according to claim 1 or 2, wherein
each of the host apparatuses comprises an examiner camera that is configured to capture images of the examiner,
the ophthalmic apparatus comprises a subject monitor that is viewable by a subject and a subject camera that is configured to capture images of the subject,
each of the host apparatuses is configured to cause the examiner monitor to display image-capture information obtained by the subject camera, and
the ophthalmic apparatus is configured to cause image-capture information obtained by the examiner camera of at least one of the plurality of host apparatuses to be displayed on the subject monitor.

5. The ophthalmic system according to claim 1 or 2, wherein the ophthalmic apparatus is configured to be remotely operated based on operation information output from one of the plurality of host apparatuses, and during the remote operation, to be prevented from receiving operation information output from the other host apparatuses.

6. The ophthalmic system according to claim 1 or 2, wherein
the plurality of host apparatuses and a plurality of ophthalmic apparatuses are configured to communicate with one another via a communication managing portion, and
the communication managing portion is configured to enable one of the plurality of ophthalmic apparatuses to be remotely operated based on operation information output from one of the plurality of host apparatuses and to enable results of eye examinations conducted by the plurality of ophthalmic apparatuses to be viewable by the plurality of host apparatuses.

7. The ophthalmic system according to claim 6, wherein the communication managing portion is configured to enable one of the plurality of host apparatuses to perform remote operation in an order of request signals output from the plurality of ophthalmic apparatuses.

## Amended claims

### Amended claims under Art. 19.1 PCT

1. (amended) An ophthalmic system, comprising:
an ophthalmic apparatus that is used in an eye examination, the ophthalmic apparatus comprising:
a subject microphone that is configured to capture audio;
a subject speaker that is configured to output audio;
a subject monitor that is viewable by a subject, and
a subject camera that is configured to capture images of the subject; and
a plurality of host apparatuses that are operated by an examiner, the plurality of host apparatuses comprising:
an examiner microphone that is configured to capture audio;
an examiner speaker that is configured to output audio;
an examiner camera that is configured to capture images of the examiner;
and
an examiner monitor that is viewable by the examiner;
wherein
the ophthalmic apparatus is provided at a remote location away from the plurality of host apparatuses, and is configured to communicate with the plurality of host apparatuses,
each of the host apparatuses is configured to communicate with other host apparatuses, and
each of the host apparatuses is configured to
cause a result of the eye examination to be displayed on the examiner monitor,
cause audio captured by the subject microphone and audio captured by an examiner microphone of the other host apparatuses to be output through the examiner speaker,
cause audio captured by the examiner microphone to be output through an examiner speaker of the other host apparatuses, and
cause image-capture information obtained by the subject camera and image-capture information obtained by the examiner camera of the other host apparatuses to be displayed on the examiner monitor, and
the ophthalmic apparatus is configured to cause audio captured by the examiner microphone to be output through the subject speaker and cause image-capture information obtained by the examiner camera of at least one of the plurality of host apparatuses to be displayed on the subject monitor.

2. (cancelled)

3. (amended) The ophthalmic system according to claim 1, wherein the subject speaker is configured to restrict the output of the audio captured by the examiner microphone when the audio is output through the examiner speaker of the other host apparatuses.

4. (cancelled)

5. (amended) The ophthalmic system according to claim 1, wherein the ophthalmic apparatus is configured to be remotely operated based on operation information output from one of the plurality of host apparatuses, and during the remote operation, to be prevented from receiving operation information output from the other host apparatuses.

6. (amended) The ophthalmic system according to claim 1, wherein
the plurality of host apparatuses and a plurality of ophthalmic apparatuses are configured to communicate with one another via a communication managing portion, and
the communication managing portion is configured to enable one of the plurality of ophthalmic apparatuses to be remotely operated based on operation information output from one of the plurality of host apparatuses and to enable results of eye examinations conducted by the plurality of ophthalmic apparatuses to be viewable by the plurality of host apparatuses.

7. The ophthalmic system according to claim 6, wherein the communication managing portion is configured to enable one of the plurality of host apparatuses to perform remote operation in an order of request signals output from the plurality of ophthalmic apparatuses.
